**Europäisches Patentamt**

**European Patent Office**

**Office européen des brevets**

(19)

(11) Veröffentlichungsnummer: **0 320 895**

**A1**

(12) **EUROPÄISCHE PATENTANMELDUNG**

(21) Anmeldenummer: **88120871.4**

(22) Anmeldetag: **14.12.88**

(51) Int. Cl.4: **A61F 5/445**

(30) Priorität: **18.12.87 DE 3743003**

(43) Veröffentlichungstag der Anmeldung:
**21.06.89 Patentblatt 89/25**

(84) Benannte Vertragsstaaten:
**AT BE CH DE ES FR GB LI LU NL SE**

(71) Anmelder: **B. Braun Melsungen AG**
**Carl-Braun Strasse**
**D-3508 Melsungen(DE)**

(72) Erfinder: **Brethauer, Ulrich**
**Rhönstrasse 2**
**D-3501 Körle(DE)**

(74) Vertreter: **von Kreisler, Alek, Dipl.-Chem. et al**
**Patentanwälte Von Kreisler-Selting-Werner**
**Deichmannhaus am Hauptbahnhof**
**D-5000 Köln 1(DE)**

(54) **Entsorgungsvorrichtung für Colostomieträger.**

(57) Die Entsorgungsvorrichtung für Colostomieträger ist aus zwei Schichten aufgebaut, nämlich einer ersten wasserunlöslichen Schicht (1), einer zweiten Schicht (2) aus einem wasserlöslichen, natürlichen oder synthetischen Polymermaterial, und einem Umbeutel (3) aus nicht wasserlöslichem, hydrophilem, natürlichem oder synthetischem Material, der mit dem Innenteil trennbar verbunden ist. Der Beutel eignet sich zum Auffangen menschlicher Ausscheidungen, wobei man den Innenbeutel solcherart verarbeitet, daß die erste wasserunlösliche Schicht (1) die Innenseite des Beutels bildet und der Umbeutel (3) aus nicht wasserlöslichem, hydrophilem, natürlichem oder synthetischem Material, einem Zellstoff, Kraftpapier, einem textilen Gewebe oder einem ungewebten Faservlies an der Außenseite des Innenbeutels liegt. Die die Außenseite des Beutels bildende dritte Schicht (3) aus hydrophilem Material ermöglicht bei Berührungskontakt mit dem Körper die Hautatmung und verhindert übermäßige Schweißbildung. Nach Gebrauch des Innenteils des Beutels kann dieser mit seinem Inhalt problemlos in die Kanalisation geleitet werden. Die Dicke der wasserunlöslichen Innenschicht kann in Abhängigkeit vom hierfür verwendeten Material so eingestellt werden, daß diese Schicht durch geringste mechanische Einwirkung beim Fortspülen zerreißt.

FIG. 7

## Entsorgungsvorrichtung für Colostomieträger

Die vorliegende Erfindung betrifft Entsorgungsvorrichtungen für Colostomieträger mit einem mehrschichtigen, im wäßrigen Medium desintegrierenden Innenbeutel. Die Entsorgungsvorrichtung für Colostomieträger dient zur Aufnahme von menschlichen Ausscheidungen. Nach Gebrauch läßt sich der im wäßrigen Medium desintegrierende Innenbeutel problemlos und umweltfreundlich beseitigen, wobei die Abfallmenge des Behälters auf ein Minimum reduziert wird.

Nach Angaben in medizinischen Fachzeitschriften gibt es zur Zeit in der Bundesrepublik Deutschland ca. 100 000 Menschen mit künstlichem Darmausgang (Ostomie). Man unterscheidet bei der Ostomie folgende Arten:
Colostomie = Dickdarmausgang
Ileostomie = Dünndarmausgang
Ureostomie = Blasenausgang.

Da die Betroffenen nicht mehr die Möglichkeit haben, die Ausscheidungen von Stuhl, Darmschleim, Sekreten und Harn willentlich zu kontrollieren, ist eine Versorgung mit einem Auffang-(Beutel)-system erforderlich.

Das zur Aufnahme von Darminhalten benötigte System (Stomaversorgung) umfaßt Beutel, Befestigungsbandagen, Hautschutz- und Abdichtmittel.

Die derzeit verwendeten Auffangbeutel werden vorwiegend aus thermoplastischen Kunststoffen wie Polyethylen, Polyvinylchlorid weich und Ethylenvinylacetatcopolymeren hergestellt und sollen absolut flüssigkeitsdicht, geruchsdicht, hautverträglich, knisterfrei, verklebbar mit Haftpflaster, unauffällig zu tragen und mit Inhalt leicht nach dem Tragen zu beseitigen sein.

Bei der Beutelbeseitigung kommt es für Colostomie-Träger mit den herkömmlichen, auf dem Markt befindlichen Beuteln zu Problemen. Ein gefüllter Beutel kann nicht in die Toilette gegeben werden, da wegen der Wasserunlöslichkeit der thermoplastischen Kunststoffe unweigerlich Verstopfungen im Kanalsystem die Folge wären.

Bisher geschieht die Beutelentsorgung entweder über den Hausmüll oder der Beutel wird in das Toilettenbecken ausgestreift und anschließend mit einer Schere in Teile geschnitten, die sich dann fortspülen lassen. Außerhalb der Wohnung, z.B. auf Reisen, gestaltet sich die Beutelentsorgung schwieriger. Oft wird ein gefüllter Beutel in Zeitungspapier oder eine Folie eingewickelt und mitgeführt, bis sich eine geeignete Möglichkeit zum Beseitigen bietet.

In der EP-PS 0 010 171 ist eine mehrschichtige, im wäßrigen Medium desintegrierende Folie und ein aus dieser Folie hergestellter Behälter und Beutel beschrieben.

Die dort beschriebene Folie besteht im wesentlichen aus einer wasserbeständigen Schicht (1), einer weiteren damit verbundenen Schicht (2) aus einem wasserlöslichen, natürlichen oder synthetischen Polymeren und einer mit diesen beiden Schichten verbundenen, desintegrierbaren dritten Schicht (3) aus nicht wasserlöslichen, hydrophilen, natürlichen oder synthetischen Polymeren.

Die dritte Schicht (3), die im fertigen Behälter als außen liegende Schicht anzusehen ist, ist vorzugsweise aus Zellstoff oder Natronkraftpapier ausgebildet. Dies hat den Vorteil, daß die wasserlösliche Schicht (2) zum Beispiel Polyvinylalkohol nicht vorzeitig durch Körperschweiß oder durch von außen durch die Kleidung dringendes Regenwasser angelöst wird. Weiterhin erhöht sich der Tragekomfort, wenn die wasserlösliche Kunststoffschicht nicht direkt mit der Hautoberfläche in Kontakt steht.

Allerdings ergibt sich in Hinblick auf das ansteigende Umweltschutzbewußtsein auch der Nachteil der zusätzlichen mengenmäßigen Entsorgung der Papierschicht nach einmaligem Gebrauch des Colostomiebeutels.

Zum anderen wird die mehrschichtige Folie durch die zusätzliche Papierschicht (3) in ihrem Verbund dicker und steifer, wobei gerade bei Colostomiebeuteln eine gute Flexibilität der Folie grundsätzlich wünschenswert ist.

Weiterhin bedingt die Papierschicht, daß das z.B. in kaltwasserlöslichen Polyvinylalkoholfolien (Schicht 2) vorhandene Wasser durch die in Kontakt stehende Papierschicht (3) tendenziell vorzeitig durch erhöhte Permeation entzogen wird. Als Resultat erhält man eine steifere und im Gebrauch auch nicht mehr geräuschfreie Folie bzw. daraus hergestellte Beutel.

Die GB-A-2 083 762 beschreibt ebenfalls Colostomiebeutel mit einem zweischichtigen Aufbau, bei dem die Enden der Folienbahnen fest miteinander verbunden sind, so daß dieselben Probleme wie bei den Colostomiebeuteln entsprechend EP-PS 0 010 171 auftreten. Der aus dem zweischichtigen Material hergestellte Colostomiebeutel kann in einer besonderen Ausführungsform in eine Einstecktasche verbracht werden, die mit Hilfe eines Bandes oder Gürtels um den Körper des Patienten mit Hilfe eines Clips gehalten werden kann. Es wird somit keinesfalls verhindert, daß die wasserlösliche Außenseite des Innenbeutels in Kontakt mit dem Körper des Patienten kommen kann.

Die vorliegende Erfindung stellt sich die Aufgabe, Entsorgungsvorrichtungen für Colostomieträger zu schaffen, die einerseits in wäßrigen Medien desintegrierbar oder zerstörbar und daher umweltfreundlich sind und zum anderen absolut flüssigkeitsdicht, geruchsdicht, hautverträglich, knisterfrei und mit Haftpflastern am Stoma fixierbar sind, wobei die Abfallmenge des Behälters auf ein Minimum reduziert ist und das im Stand der Technik vorhandene Problem gelöst wird, daß die wasserlösliche Außenseite des Innenbeutels in Kontakt mit dem Körper des Patienten geraten kann.

Diese Aufgaben werden erfindungsgemäß durch die zur Verfügung gestellten Entsorgungsvorrichtungen gelöst.

Die erfindungsgemäßen Entsorgungsvorrichtungen bestehen aus einem Colostomiebeutel, bestehend aus einem Innenbeutel aus einer im wäßrigen Medium desintegrierenden zweischichtigen Folie mit einer die Innenseite des Beutels bildenden ersten wasserbeständigen Schicht (1) und einer weiteren, damit verbundenen zweiten Schicht (2) aus einem wasserlöslichen, natürlichen oder synthetischen Polymeren und einem Umbeutel (3), bestehend aus wiederverwendbarem, nicht wasserlöslichem hydrophilen, natürlichem oder synthetischem Polymeren, einem textilen Gewebe, einem ungewebten Faservlies oder aus Kraftpapier, wobei der Umbeutel (3) den Innenbeutel zumindest teilweise umhüllt und nach Austausch des Innenbeutels wiederverwendbar ist und wobei in der Wand des Beutels eine Öffnung (5) zur Einleitung der menschlichen Ausscheidungen vorgesehen ist, dadurch gekennzeichnet, daß an der zweiten Schicht (2) eine die Öffnung (5) ringförmig umgebende, selbstklebende Schicht (7) befestigt ist und der Umbeutel (3) eine Öffnung (12) aufweist, deren Durchmesser zwischen dem Durchmesser der Öffnung (5) im Innenbeutel (1, 2) und dem Außendurchmesser der selbstklebenden Schicht (7) des Innenbeutels liegt.

Das Konzept der vorliegenden Erfindung besteht darin, daß man, ausgehend von der EP-PS 010 171, unter Beibehaltung der wasserbeständigen Schicht (1) und der damit verbundenen zweiten Schicht (2) aus einem wasserlöslichen natürlichen oder synthetischen Polymeren die dritte Schicht (3) trennbar anordnet, so daß der daraus gebildeten Umhüllung (3) ein darin befindlicher Colostomiebeutel entnommen werden kann.

Die sehr dünne, wasserunlösliche Schicht (1) stellt wiederum das Siegelmedium bei der Weiterverarbeitung des Folienverbundes zu Colostomiebeuteln dar. Die wasserlösliche Außenschicht (2) gibt dem Beutel genügend Stabilität und dient gleichzeitig als optimale Geruchsbarriere.

Die auf diese Weise hergestellte Verbundfolie ist sehr weich und flexibel, wodurch der Colostomiebeutel eine gute Dehnfähigkeit erhält und beim Tragen keine Knistergeräusche erzeugt.

Zum Schutz der wasserlöslichen Beutelaußenseite dient ein flauschiger, Wasser- und Körperschweiß-abstoßender Umbeutel (3), der gleichzeitig den Tragekomfort erhöht und mehrfach verwendbar ist. Eine Aussparung (5) auf der Umbeutelrückseite ermöglicht die funktionsfähige, paßgenaue Kombination des am Colostomiebeutel befindlichen Klebepflasters (7) mit dem Stoma, damit die aus Colostomiebeutel und Umhüllung (3) bestehende Entsorgungsvorrichtung in geeigneter Weise auf die Haut der Bauchdecke aufgeklebt werden kann.

Als Umbeutelmaterial (3) hat sich ein Faservlies, z. Beispiel auf Polypropylenfaserbasis als günstig herausgestellt. Das Material ist schweißbar, hat eine gute Festigkeit und erfüllt die oben genannten Eigenschaften.

Für die Herstellung der Umbeutel (3) können aber auch andere Stoffmaterialien, beispielsweise genähte Textilbeutel mit wasseraufsaugender Wirkung verwendet werden.

Gegebenenfalls können auch Cellulosepapier in Tissuequalitäten, Kraftpapier oder ähnliche Materialien natürlichen oder synthetischen Ursprungs mit glatten oder auch strukturierten Oberflächen verwendet werden.

Entscheidend für die Auswahl des Materials ist insbesondere der Tragekomfort sowie die Haltbarkeit und gegebenenfalls die Waschbarkeit dann, wenn der Innenteil, bestehend aus einer zweischichtigen Folie, entnehmbar ist.

Der Umbeutel kann in gewissem Umfange wasserabweisenden Charakter haben oder wasserabweisend ausgerüstet sein.

Die erste wasserbeständige und wasserunlösliche Schicht (1) besteht z.B. aus einer siegelbaren, synthetischen Schmelzkleberkomposition, einem siegelbaren, synthetischen (Siegel)Lack, einer thermoplastischen Folie, einer Elastomerfolie oder einem wasserbeständigen Papier aus natürlichen oder synthetischen Rohstoffen. Diese erste wasserunlösliche Schicht kann eine Dicke von 2 bis 20 $\mu$m, vorzugsweise von 10 bis 15 $\mu$m, aufweisen.

Thermoplastische Folien der nicht wasserlöslichen Schichten können aus z.B. Polyethylen-, Polypropylen-, Polyamid-, oder Polyesterhomo- oder Copolymerisaten aufgebaut werden. Siegelbare bzw. verschweißbare Elastomere werden auf Basis Styrol-Butadien-Styrol oder Styrol-Isopren-Styrol oder auf Ethylen-Propylen-Kautschuk (EPDM) modifizierten Polyethylenhomo- oder Copolymerisaten eingesetzt.

Wasserbeständige, papierähnliche Bahnen werden z.B. aus Polyethylenendlosfasern oder aus einer Polyethylenpulpe hergestellt.

Die Schicht (2) besteht aus einem wasserlöslichen Material natürlicher, teilsynthetischer oder synthetischer Herkunft. Beispiele für geeignete Materialien sind Polyvinylalkohol, Polyethylenoxid, Hydroxyethylcellulose, Carboxymethylcellulose, Natriumalginat, Polyacrylsäure, Polyacrylamid, Polyethylenimin, Polyvinylpyrrolidon, Stärke und Gelatine.

Bevorzugt werden als Materialien Polyvinylalkohol oder teilweise verseiftes Polyvinylacetat.

Diese wasserlösliche Schicht (2) kann eine Dicke von 10 bis 100 μm, vorzugsweise von 40 bis 60 μm, aufweisen.

Bei einer erfindungsgemäßen Entsorgungsvorrichtung ergeben sich allgemein verschiedene Probleme. Sie kann nicht nur aus wasserlöslichen Werkstoffen bestehen, da die Innenseiten des Beutels bereits durch die Körperausscheidungen, die im Falle von Urin völlig wäßrig sind, bei Faeces einen Bereich geringer Feuchtigkeit bis weitgehend wäßrig umfassen können, angelöst bzw. die gesamte Beutelwand stellenweise aufgelöst werden, wodurch der gesamte Beutel augenblicklich undicht und somit unbrauchbar würde.

Weiterhin ist es wahrscheinlich, daß die Außenseite des Innenteils eines solchen Beutels durch Schweißeinwirkung oder in die Kleidung eingedrungenes Regenwasser angelöst wird, was ebenfalls zu Undichtigkeiten oder einer Zerstörung führen würde.

Das Problem besteht also darin, einen einerseits von innen völlig flüssigkeitsdichten und geruchsdichten Beutel und andererseits eine von außen mit der Haut verträglichen und vorzugsweise undurchsichtige Umhüllung (3) herzustellen, wobei der Innenbeutel leicht in den üblichen Abwässerentfernungsanlagen zerstörbar ist, wobei die Abfallmenge auf ein Minimum reduziert wird.

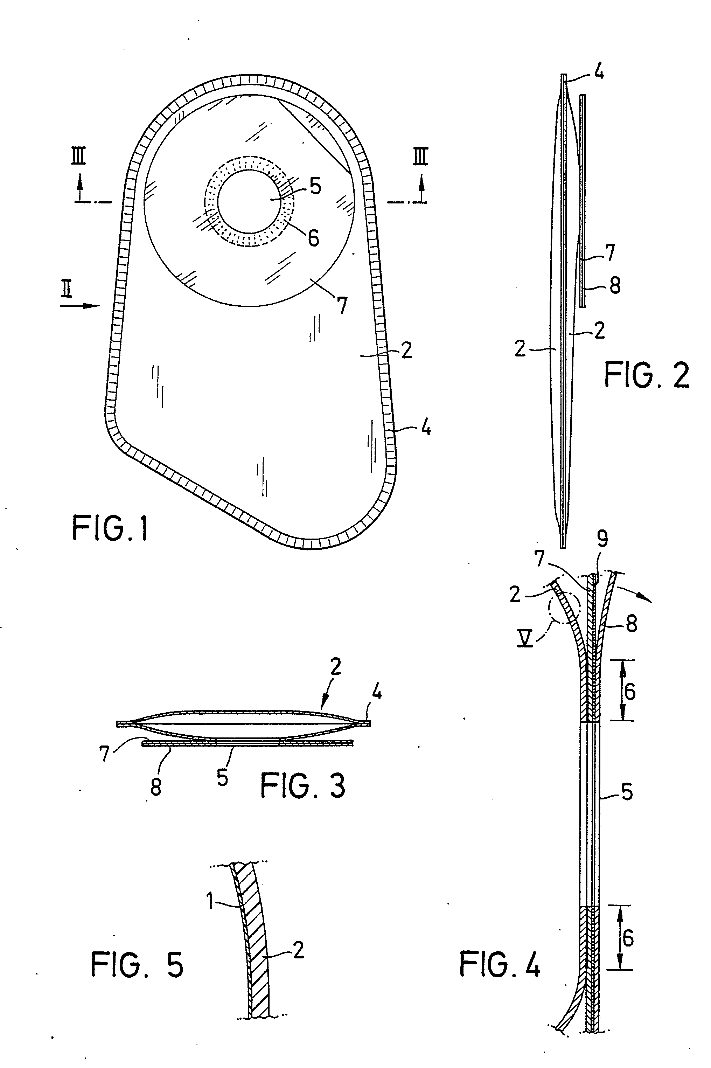

Dies wird dadurch erreicht, daß die Entsorgungsvorrichtung den erfindungsgemäßen Aufbau, wie in den Figuren 1 bis 7 beschrieben, aufweist. Dabei ist die Innenseite des Beutels, die mit den Ausscheidungen in Kontakt kommt, die erste wasserunlösliche Schicht (1) der Folie, die z.B. in Form eines Lackes, Schmelzklebers, eines wasserbeständigen Papiers, einer Thermoplastfolie oder Elastomerfolie vorliegt.

Diese erste Schicht (1) - oder im Falle des Beutels Innenschicht - ist einerseits genügend fest, um abso lut flüssigkeitsdicht und geruchsdicht zu sein, aber zum anderen mechanisch zerstörbar, so daß eine Desintegration möglich ist.

Auf dieser ersten inneren Schicht (1) ist die zweite Schicht (2) aus wasserlöslichem polymerem Material auflaminiert.

Diese zweite Schicht (2) ist mit dem Umbeutel (3) zerstörungsfrei trennbar verbunden. Dieser Umbeutel der Entsorgungsvorrichtung besteht z.B. aus einer Zellstoffschicht, einem textilen Gewebe, einem ungewebten Faservlies oder Kraftpapier, so daß der Innenbeutel entnehmbar ist. Die Eigenschaft, daß die zweite Schicht (2) und der Umbeutel (3) zerstörungsfrei trennbar miteinander verbunden sind, bedeutet im Sinne der vorliegenden Erfindung, daß diese beiden Schichten lose aufeinander liegen und die Trennung der beiden Schichten durch den Benutzer des Colostomiebeutels möglich ist.

Die feuchtigkeitsundurchlässige erste oder innere Schicht (1) sollte eine geringe Wanddicke haben. Beispielsweise kommen Dicken von 2 bis 20 μm, vorzugsweise von 10 bis 15 μm, in Frage.

Die Beutelaußenseite ist somit gegen äußere Feuchtigkeitseinwirkung oder Körperschweiß ausreichend isoliert. Der eine derartige Verbundfolie enthaltende Beutel kann dem Umbeutel (3) entnommen werden und direkt in die Toilettenschüssel, vorzugsweise in die Ablauföffnung, die bei allen Toilettenbecken immer mit Wasser gefüllt ist, gegeben werden, wobei die Abfallmenge auf ein Minimum reduziert ist.

Der Auflösevorgang vollzieht sich dann schrittweise. Zuerst wird die wasserlösliche Schicht (2) aufgelöst. Ungelöst bleibt die innere wasserundurchlässige Schicht (1) in Form eines dünnen Films, der wegen der geringen Wanddicke keine nennenswerte mechanische Festigkeit hat. Beim Spülvorgang im Toilettenbecken reißt dieser verbliebene, weil wasserunlösliche und die Ausscheidung noch umhüllende Folienfilm durch mechanische Einwirkungen im Ablaufsystem ein und alle Komponenten können problemlos in die Kanalisation geleitet werden.

Die Entsorgungsvorrichtung gemäß der Erfindung bietet noch weitere Vorteile gegenüber herkömmlichen Beutelsystemen aus thermoplastischen Folienmaterialien. Es ist bekannt, daß bei unmittelbarem Kontakt der Haut mit thermoplastischen wasserundurchlässigen Folien es besonders in diesem Bereich wegen behinderter Hautatmung zu Schweißabsonderungen kommt, was als unangenehm empfunden wird. Wählt man bei direktem Kontakt mit der Haut eine atmungsaktive Schicht, kann die Haut besser atmen. Schweißabsonderungen werden dadurch vermieden. Ebenfalls wird die Sicht auf den Beutelinhalt versperrt. Beutel aus transparenten Folien dagegen würden einen unangenehmen Anblick bieten.

Weiterhin ist bekannt, daß Polyvinylalkoholfolien - im Gegensatz zu Folien aus Polyethylen und Polyvinylchlorid - eine extrem niedrige Gas- und Aromadurchlässigkeit besitzen. Die übelriechenden Darmgase können den Patienten und dessen Umgebung nicht mehr belästigen, was bei sozialen Begegnungen auch ein äußerst wichtiger psychosozialer Faktor ist. Polyvinylalkoholfolie ist physiologisch unbedenklich, zeigt in Abwasser keine phytotoxische Wirkung und ist in Kläranlagen mit adaptierter biologischer Klärstufe abbaubar.

Gemäß einer Ausführungsform der vorliegenden Erfindung ist die Entsorgungsvorrichtung für Colostomieträger derart aufgebaut, daß der Innenbeutel (1, 2) und der Umbeutel (3) zur Erleichterung des Ineinandersetzens trapezförmig verlaufende Seitenränder aufweisen.

In einer weiteren Ausführungsform der vorliegenden Erfindung ist die Entsorgungsvorrichtung dadurch gekennzeichnet, daß der Umbeutel (3) eine Öffnung (12) aufweist, deren Durchmesser zwischen dem Durchmesser der Öffnung (5) im Innenbeutel (1, 2) und dem Außendurchmesser der selbstklebenden Schicht (7) des Innenbeutels liegt.

Vorzugsweise ist die Entsorgungsvorrichtung für Colostomieträger der vorliegenden Erfindung dadurch gekennzeichnet, daß sich an die Öffnung (12) im Umbeutel (3) an zwei einander gegenüberliegenden Stellen Schlitze (13) zur Erleichterung des Hindurchführens der selbstklebenden Schicht (7) des Innenbeutels (1, 2) durch die Öffnung (12) im Umbeutel (3) nach dem Einsetzen des Innenbeutels in den Umbeutel anschließen.

Die Erfindung wird anhand der Figuren 1 bis 7 weiter erläutert:

Die Figur 1 zeigt den Innenbeutel der erfindungsgemäßen Entsorgungsvorrichtung für Colostomieträger mit der die Außenschicht bildenden wasserlöslichen Schicht (2). Die Oberseite und die Unterseite des Innenbeutels sind durch die umlaufende Schweißnaht (4) miteinander verbunden. Der Beutel zum Auffangen der menschlichen Ausscheidungen weist eine Öffnung (5) auf, die zur Einleitung der menschlichen Ausscheidungen vorgesehen ist. Um die Öffnung (5) herum ist der Auffangbeutel im Bereich des Kreisringes (6) mit einer ringförmigen Klebevorrichtung (7) versehen, die das Anbringen der erfindungsgemäßen Entsorgungsvorrichtung gestattet.

Die Figur 2 stellt die Seitenansicht der Figur 1 entlang der Blickrichtung II dar und zeigt die mit der umlaufenden Schweißnaht (4) verbundenen desintegrierbaren Schichten sowie die die Außenwand bildende wasserlösliche Schicht (2). Weiterhin ist der Figur 2 der Aufbau der Klebevorrichtung (7) zu entnehmen, die mit einer abziehbaren Abdeckeinrichtung versehen ist.

Die Figur 3 zeigt den Querschnitt des Innenbeutels entlang der Blickrichtung III gemäß Figur 1.

Die Figur 4 zeigt die Anbringung der Klebeeinrichtung (7). Im Bereich des Kreisringes (6) ist die Klebeeinrichtung (7) um die Öffnung (5) mit der wasserlöslichen Schicht (2) verbunden. Auf der Klebeeinrichtung (7) befindet sich eine Klebstoffschicht (9), die nach Entfernen der abziehbaren Schicht (8) das Aufsetzen der Entsorgungsvorrichtung erlaubt.

Die Figur 5 zeigt die Vergößerung aus V, Figur 4 und somit den zweischichtigen Aufbau des Innenbeutels mit der desintegrierbaren Schicht (1) und der darauf laminierten wasserlöslichen Schicht (2), die die Außenseite des Innenbeutels bildet.

Die Figur 6 zeigt den Innenbeutel mit der wasserlöslichen Schicht (2), der durch Einschieben in Pfeilrichtung in den Umbeutel (3) eingeführt wird.

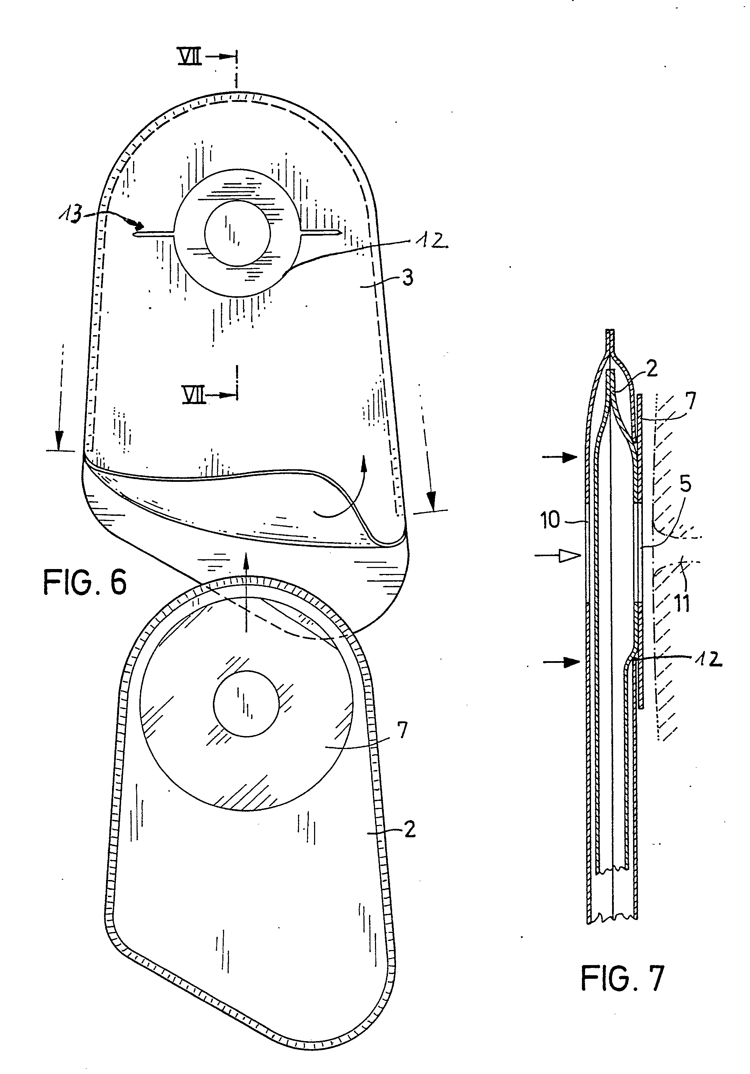

Weiterhin ist der Figur 6 zu entnehmen, daß der Innenbeutel (2) und der Umbeutel (3) zur Erleichterung des Ineinandersetzens trapezförmig verlaufende Seitenränder enthalten.

Weiterhin weist der Umbeutel (3) eine Öffnung (12) auf, deren Durchmesser zwischen dem Durchmesser der Öffnung (5) im Innenbeutel (2) und dem Außendurchmesser der selbstklebenden Schicht (7) des Innenbeutels (2) liegt.

Die sich an die Öffnung (12) im Umbeutel (3) an zwei einander gegenüberliegenden Stellen befindlichen Schlitze (13) erleichtern das Hindurchführen der selbstklebenden Schicht (7) des Innenbeutels (2) durch die Öffnung (12) im Umbeutel nach dem Einsetzen des Innenbeutels in den Umbeutel.

Die Figur 7 zeigt ein Sichtfenster (10) im Umbeutel (3), das so angebracht ist, daß beim Aufsetzen der Entsorgungsvorrichtung das Stoma beobachtet werden kann.

Die Herstellung der erfindungsgemäßen mehrschichtigen Verbundfolie erfolgt nach bekannten Verfahren zur Herstellung von laminierten Folien.

Ein geeignetes Verfahren zur Herstellung der Verbundfolie:

Auf die als Bahn vorgesehene zweite Schicht aus Polyvinylalkohol wird durch Walzenauftrag oder über eine Breitschlitzdüse die wasserunlösliche, siegelbare erste Schicht extrudiert.

Der Aufbau der erfindungsgemäßen Entsorgungsvorrichtungen wird in den folgenden Beispielen geschildert.

Beispiel 1

Die erste, innenliegende Schicht (1) besteht aus einer nach bekannten Schweißverfahren siegelbaren, nicht wasserlöslichen Polymerfolie aus thermoplastischem Polyethylen niedriger Dichte in einer Folienstärke von 15 μm. Als Trägerschicht (2) dient eine 50 μm dicke Folie aus Polyvinylalkohol oder teilweise verseiftem Polyvinylacetat, die in einer einstellbaren Zeit in Wasser auflösbar ist. Der Umbeutel besteht aus nicht gewebtem Faservliesmaterial auf Polypropylenbasis.

Beispiel 2

Als erste Schicht (1) wird ein handelsüblicher hot melt-Kleber (Schmelzkleber) in einer Dicke von 10 μm verwendet; die Trägerschicht (2) besteht aus Polyethylenoxidfolie mit 60 bis 80 μm Dicke.

Der Umbeutel (3) besteht aus non woven fabric auf Polypropylenfaserbasis.

## Ansprüche

1. Entsorgungsvorrichtung für Colostomieträger aus einem Colostomiebeutel, bestehend aus einem Innenbeutel aus einer im wäßrigen Medium desintegrierenden zweischichtigen Folie mit einer die Beutelinnenseite bildenden ersten wasserbeständigen Schicht (1) und einer weiteren, damit verbundenen zweiten Schicht (2) aus einem wasserlöslichen, natürlichen oder synthetischen Polymeren und einem Umbeutel (3), bestehend aus wiederverwendbarem, nicht wasserlöslichem hydrophilen, natürlichem oder synthetischem Polymeren, einem textilen Gewebe, einem ungewebten Faservlies oder aus Kraftpapier, wobei der Umbeutel (3) den Innenbeutel zumindest teilweise umhüllt und nach Austausch des Innenbeutels wiederverwendbar ist und wobei in der Wand des Beutels eine Öffnung (5) zur Einleitung der menschlichen Ausscheidungen vorgesehen ist, dadurch gekennzeichnet, daß an der zweiten Schicht (2) eine die Öffnung (5) ringförmig umgebende, selbstklebende Schicht (7) befestigt ist und der Umbeutel (3) eine Öffnung (12) aufweist, deren Durchmesser zwischen dem Durchmesser der Öffnung (5) im Innenbeutel (1, 2) und dem Außendurchmesser der selbstklebenden Schicht (7) des Innenbeutels liegt.

2. Entsorgungsvorrichtung für Colostomieträger mit einem Colostomiebeutel nach Anspruch 1, dadurch gekennzeichnet, daß die erste, wasserunlösliche Schicht (1) aus einer siegelbaren synthetischen Schmelzkleberkomposition, einem siegelbaren, synthetischen Siegellack, einer Thermoplastfolie, einer Elastomerfolie oder einem wasserbeständigen Papier besteht.

3. Entsorgungsvorrichtung für Colostomieträger mit einem Colostomiebeutel nach Ansprüchen 1 und 2, dadurch gekennzeichnet, daß die erste, wasserunlösliche Schicht (1) eine Dicke von 2 bis 20 μm, vorzugsweise von 10 bis 15 μm, aufweist.

4. Entsorgungsvorrichtung für Colostomieträger mit einem Colostomiebeutel nach Anspruch 1, dadurch gekennzeichnet, daß die zweite, wasserlösliche Schicht (2) aus Polyvinylalkohol oder teilweise verseiftem Polyvinylacetat besteht.

5. Entsorgungsvorrichtung für Colostomieträger mit einem Colostomiebeutel nach Ansprüchen 1 bis 4, dadurch gekennzeichnet, daß die zweite wasserlösliche Schicht (2) eine Dicke von 10 bis 100 μm, vorzugsweise von 40 bis 60 μm, aufweist.

6. Entsorgungsvorrichtung für Colostomieträger nach Ansprüchen 1 bis 5, dadurch gekennzeichnet, daß der Umbeutel (3) mit einer zusätzlichen Öffnung (10) zur Beobachtung des Stomas beim Aufsetzen der Entsorgungsvorrichtung durch die Öffnung (5) versehen ist.

7. Entsorgungsvorrichtung für Colostomieträger nach Ansprüchen 1 bis 6, dadurch gekennzeichnet, daß sie zur Einleitung menschlicher Ausscheidungen mit einem ableitenden Katheter verbunden ist.

8. Entsorgungsvorrichtung nach Ansprüchen 1 bis 7, dadurch gekennzeichnet, daß der Innenbeutel (1, 2) und der Umbeutel (3) zur Erleichterung des Ineinandersetzens trapezförmig verlaufende Seitenränder aufweisen.

9. Entsorgungsvorrichtung nach Ansprüchen 1 bis 8, dadurch gekennzeichnet, daß sich an die Öffnung (12) im Umbeutel (3) an zwei einander gegenüberliegenden Stellen Schlitze (13) zur Erleichterung des Hindurchführens der selbstklebenden Schicht (7) des Innenbeutels (1, 2) durch die Öffnung (12) im Umbeutel (3) nach dem Einsetzen des Innenbeutels in den Umbeutel anschließen.

FIG.1

III

III

II

5

6

7

2

4

FIG. 2

4

4

7

8

2

2

9

7

2

8

V

6

6

5

2

4

7

8

5

FIG. 3

1

2

FIG. 5

FIG. 4

**FIG. 6**

**FIG. 7**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 519 797 (HALL) <br> * Spalte 1, Zeilen 49-54; Spalte 2, Zeilen 24-25; Figuren 1,3 * <br> --- | 1-5,7-9 | A 61 F 5/445 |
| D,Y | GB-A-2 083 762 (ENAK) <br> * Seite 1, Zeilen 59-66,87-97 * <br> --- | 1-5,7-9 | |
| D,Y | EP-A-0 010 171 (INTERMEDICAT) <br> * Seite 7, Zeilen 1-4 * <br> --- | 3 | |
| Y | US-A-4 238 059 (CARAWAY et al.) <br> * Spalte 4, Zeilen 33-37; Figur 3 * <br> --- | 7 | |
| Y | US-A-4 219 023 (GALINDO) <br> * Spalte 3, Zeilen 30-33; Figur 1 * <br> ----- | 9 | |

| | | | RECHERCHIERTE SACHGEBIETE (Int. Cl.4) |
|---|---|---|---|
| | | | A 61 F |

Der vorliegende Recherchenbericht wurde für alle Patentansprüche erstellt

| Recherchenort | Abschlußdatum der Recherche | Prüfer |
|---|---|---|
| DEN HAAG | 15-03-1989 | MOERS R.J. |